# EUROPEAN PATENT APPLICATION

(11) **EP 2 412 350 A1**
(43) Date of publication of application: **01.02.2012**
(21) Application number: 10755873.6
(22) Date of filing: 10.03.2010
(51) Int. Cl.: A61F 13/20

(54) **TAMPON, METHOD FOR PRODUCING TAMPON, AND APPARATUS FOR PRODUCING TAMPON**

(30) Priority: 23.03.2009 JP 2009070647
(71) Applicant: Uni-Charm Corporation, Ehime 799-0111 (JP)
(72) Inventor: WADA, Mitsuhiro, Kanonji-shi Kagawa 769-1602 (JP); NOZAKI, Satoshi, Kanonji-shi Kagawa 769-1602 (JP)
(74) Representative: Vaughan, Jennifer Ann
(86) International application number: PCT/JP2010/054035
(87) International publication number: WO 2010/110073

(57) **Abstract**

A tampon has an absorbent body that absorbs liquid. On an outer surface of the absorbent body, the absorbent body includes an applied portion to which an agent is applied and that includes at least two applied layer. The at least two applied layer includes a first applied layer formed by applying a first agent, and a second applied layer formed by applying a second agent over the first applied layer, the first agent including active pharmaceutical ingredient and a first water-soluble carrier that carries the active pharmaceutical ingredient, the second agent including active pharmaceutical ingredient and a second water-soluble carrier that carries the active pharmaceutical ingredient and has a melting point lower than that of the first water-soluble carrier.

## Description

### Technical Field

The invention relates to a tampon, a manufacturing method for tampon, and a manufacturing apparatus for a tampon.

### Background Art

Tampons including an absorbent body that absorbs liquid such as menstrual blood are well known. In some such tampons, an agent is applied onto an outer surface of the absorbent body thereof.

### Citation List

### [Patent Literature]

[PTL 1] Japanese Unexamined Patent Application Publication (Translation of PCT Application) No. 2005-536237

### Summary of the Invention

### Technical Problem

There was a possibility that the agent does not properly take effect while the foregoing tampon to which the agent is applied is inserted in the vaginal cavity. Therefore, a tampon having an agent that properly takes effect has been demanded.

This invention has been made in view of the above problems, and an advantage thereof is to provide a tampon having an agent that properly takes effect while the tampon is inserted in the vaginal cavity.

### Solution to Problem

An aspect of the invention to achieve the above advantage is
a tampon having an absorbent body that absorbs liquid, including:
on an outer surface of the absorbent body,
an applied portion to which an agent is applied and that includes at least two applied layers having
a first applied layer formed by applying a first agent, and
a second applied layer formed by applying a second agent over the first applied layer,
the first agent including an active pharmaceutical ingredient and a first water-soluble carrier that carries the active pharmaceutical ingredient,
the second agent including an active pharmaceutical ingredient and a second water-soluble carrier that carries the active pharmaceutical ingredient and has a melting point lower than that of the first water-soluble carrier.

Other features of this invention will become apparent from the description in this specification and the attached drawings.

### Effects of the Invention

According to the invention, a tampon having an agent that properly takes effect while the tampon is inserted in the vaginal cavity is achieved.

### BRIEF DESCRIPTION OF THE DRAWINGS

FIG. 1 is a cross-sectional view showing components of a tampon 10.
FIG. 2 is a cross-sectional view showing components of the tampon 10.
FIG. 3A is an external view of a tampon body 20. FIG. 3B is a schematic diagram showing a state of an applied layer in an applied portion 23.
FIG. 4 is an external view of an outer tube 40.
FIG. 5 is a view of the outer tube 40 shown in FIG. 4 from its front end.
FIG. 6 is a cross-sectional view taken along line A-A in FIG. 1 or 2.
FIG. 7 is a magnified view of FIG. 2.
FIG. 8 is a flowchart showing the production flow of the tampon body 20.
FIGS. 9A to 9D are schematic diagrams showing transition of the tampon body 20 to a finished product.
FIG. 10 is a schematic diagram showing a section of the manufacturing apparatus 100 for the tampon 10, which manufactures the tampon body 20.
FIG. 11 is a schematic diagram the manufacturing apparatus shown in FIG. 10 viewed from above.
FIG. 12 is a diagram showing a pattern according to the other embodiment.
FIG. 13 is a diagram showing a pattern according to the other embodiment.

### Mode for Carrying Out the Invention

At least the following matters will be made clear by the description in the present specification and the accompanying drawings.

A tampon having an absorbent body that absorbs liquid, including:
on an outer surface of the absorbent body,
an applied portion to which an agent is applied and that includes at least two applied layers having
a first applied layer formed by applying a first agent, and
a second applied layer formed by applying a second agent over the first applied layer,
the first agent including an active pharmaceutical ingredient and a first water-soluble carrier that carries the active pharmaceutical ingredient,
the second agent including an active pharmaceutical ingredient and a second water-soluble carrier that carries the active pharmaceutical ingredient and has a melting point lower than that of the first water-soluble carrier.
In such a case, a tampon having an agent that properly takes effect while the tampon is inserted in the vaginal cavity is achieved.

In such a tampon, desirably,
the second applied layer is a layer formed by applying the second agent over the first applied layer such that the second applied layer does not exceed a boundary of the first applied layer.
In such a case, a tampon having an agent that more properly takes effect while the tampon is inserted in the vaginal cavity is achieved.

In such a tampon, desirably,
the absorbent body includes, on the outer surface thereof, the applied portion to which the agent is applied and a non-applied portion to which the agent is not applied, and
a pattern is formed by the applied portion and the non-applied portion on the outer surface.
This can definitely make the tampon appear attractive.

In such a tampon, desirably,
the first water-soluble carrier has a melting point higher than body temperature, and
the second water-soluble carrier has a melting point lower than or equal to body temperature.
In such a case, a tampon having an agent that properly takes effect while the tampon is inserted in the vaginal cavity is achieved more definitely.

A manufacturing method for a tampon having an absorbent body that absorbs liquid, including:
obtaining the absorbent body by compressing and shaping an absorbent-body material;
applying onto an outer surface of the absorbent body a first agent that is melted and includes an active pharmaceutical ingredient and a first water-soluble carrier carrying the active pharmaceutical ingredient, the outer surface having a temperature lower than or equal to a freezing point of the first water-soluble carrier; and
applying over the first agent a second agent that is melted and includes an active pharmaceutical ingredient and a second water-soluble carrier that carries the active pharmaceutical ingredient and has a melting point lower than that of the first water-soluble carrier, the first agent having a temperature lower than or equal to a freezing point of the second water-soluble carrier.
In such a case, a manufacturing method for a tampon having agents (the first agent and the second agent) that properly take effect while the tampon is inserted in the vaginal cavity is achieved.

A manufacturing apparatus for a tampon having an absorbent body that absorbs liquid, including:
a compressing-shaping unit with which the absorbent body is obtain by compressing and shaping an absorbent-body material; and
an applying unit
that applies onto an outer surface of the absorbent body a first agent that is melted and includes an active pharmaceutical ingredient and a first water-soluble carrier carrying the active pharmaceutical ingredient, the outer surface having a temperature lower than or equal to a freezing point of the first water-soluble carrier, and
that applies over the first agent the second agent that is melted and includes a active pharmaceutical ingredient and a second water-soluble carrier that carries the active pharmaceutical ingredient and has a melting point lower than that of the first water-soluble carrier, the first agent having a temperature lower than or equal to a freezing point of the second water-soluble carrier.
In such a case, a manufacturing apparatus for a tampon having agents (the first agent and the second agent) that properly take effect while the tampon is inserted in the vaginal cavity is achieved.

### === Configuration of Tampon ===

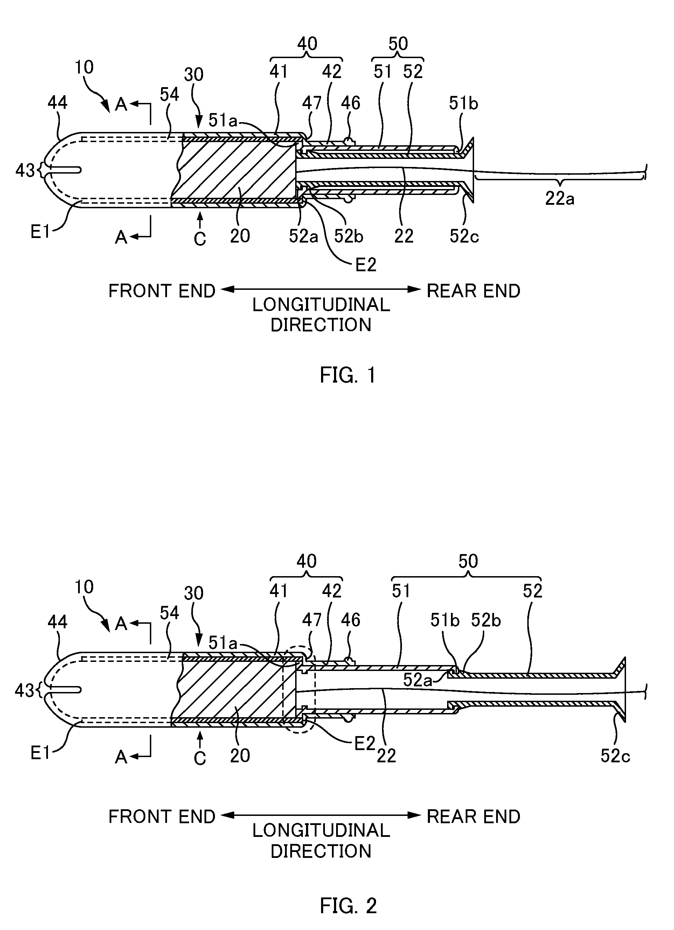

Firstly, the configuration of a tampon 10 will be described with reference to FIGS. 1 to 7.

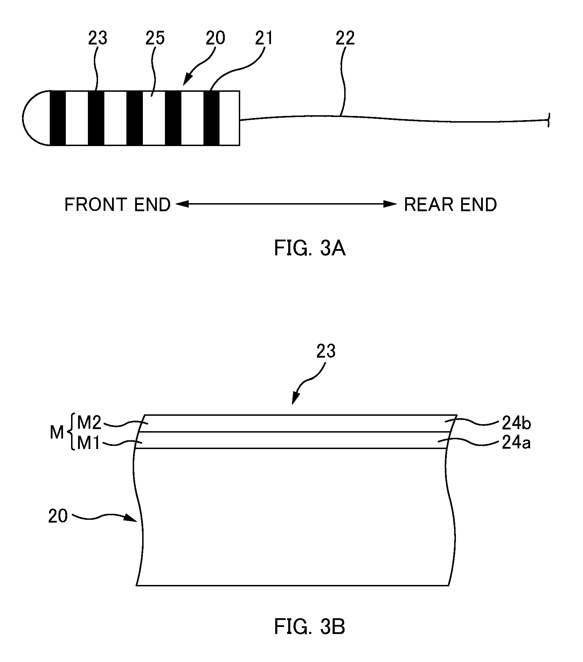

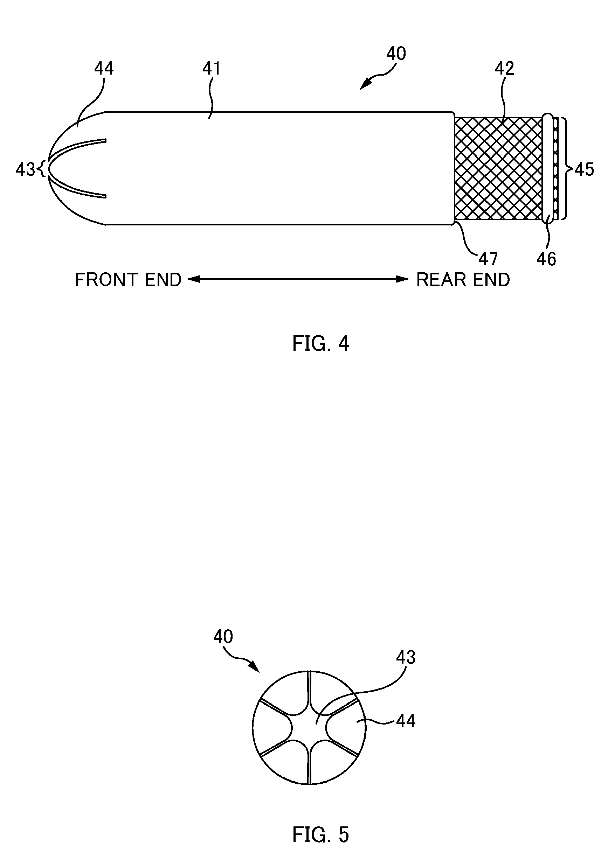

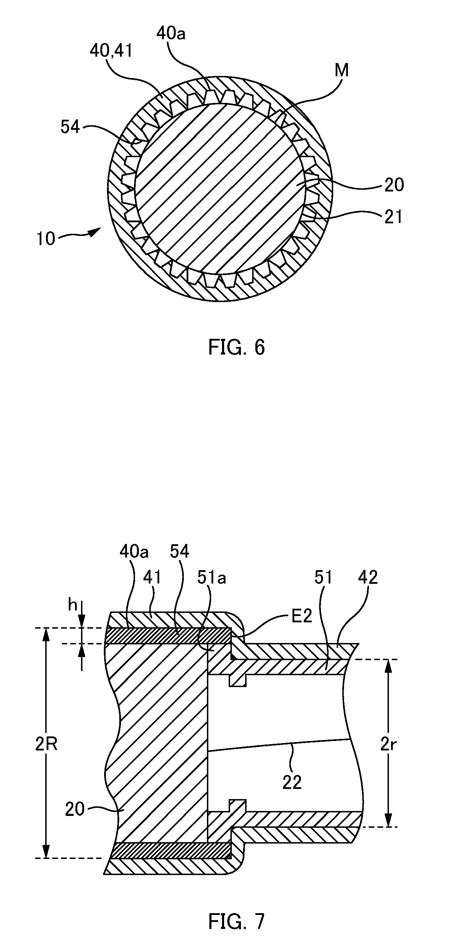

FIGS. 1 and 2 are cross-sectional views showing components of a tampon 10. FIG. 1 shows the tampon 10 with its inner tube 50 shortened, and FIG. 2 shows the tampon 10 with its inner tube 50 elongated. FIG. 3A is an external view of a tampon body 20. FIG. 3B is a schematic diagram showing a state of an applied layer in an applied portion 23. FIG. 4 is an external view of an outer tube 40. FIG. 5 is a view of the outer tube 40 shown in FIG. 4 from its front end. FIG. 6 is a cross-sectional view taken along A-A of FIGS. 1 or 2. FIG. 7 is a magnified view of FIG. 2. In the following description, in a longitudinal direction of the tampon 10, an end inserted into the vaginal cavity is referred to as a front end and the opposite end is referred to as a rear end.

As shown in FIGS. 1 and 2, the tampon 10 of the present embodiment includes the tampon body 20 as an example of the absorbent body, and an applicator 30 having the outer tube 40 as an example of housing cylinder and the inner tube 50 as an example of a pushing member.

The tampon body 20 is a thing to fill the vaginal cavity and absorb liquid such as menstrual blood. This tampon body 20 is formed by covering an absorbent main body (wool-like body) made of rayon fiber, with a cover made of polyester spunbond nonwoven fabric. The tampon body 20 is shaped substantially like a bullet.

Further, an agent M is applied onto an outer surface 21 of the tampon body 20 (applied portions 23 to which the agent M is applied are included). This agent M is a light brown colorant (a colored substance), and is a mixture of a pine bark extract (flavangenol ® manufactured by Toyo Shinyaku Co., Ltd.) and a polyethylene glycol, the pine bark extract being as an example of an active pharmaceutical ingredient that is administered to inside of the vaginal cavity and performs antioxidant activity, anti-inflammatory activity, antibiotic activity, antiviral activity, antiallergic activity, deodorization, vasodilation, inhibitory action on lipid peroxidation etc, and the polyethylene glycol being as an example of a water-soluble carrier that carries the pine bark extract.

More specifically, as shown in FIG. 3B, the applied portion 23 includes at least two applied layers (in the present embodiment, two applied layers; hereinafter referred to as a first applied layer 24a and a second applied layer 24b). The second applied layer 24b is applied over the first applied layer 24a (that is, the second applied layer 24b is located outside the first applied layer 24a). More specifically, the second applied layer 24b is a layer formed by applying the following second agent M2 over the first applied layer 24a such that the second applied layer 24b does not exceed the boundaries of the first applied layer 24a.

Further, each of the applied layers is a layer formed by applying the mixture of the pine bark extract and the polyethylene glycol. However, the polyethylene glycols of the first applied layer and the second applied layer are different types of polyethylene glycols having a different melting point (in other words, freezing point). That is, the agent M applied onto the first applied layer 24a (hereinafter referred to as a first agent M1, for convenience) includes the pine bark extract and a polyethylene glycol with a molecular weight of 1540 (hereinafter referred to as the first polyethylene glycol) as an example of the first water-soluble carrier. On the other hand, the agent M applied onto the second applied layer (hereinafter referred to as a second agent M2, for convenience) includes the pine bark extract and a polyethylene glycol with a molecular weight of 1000 (hereinafter referred to as second polyethylene glycol) as an example of the second water-soluble carrier. The melting point (freezing point) of the first polyethylene glycol is approximately 45°C, higher than body temperature (37°C). On the other hand, the melting point (freezing point) of the second polyethylene glycol is approximately 37°C, which is lower than the melting point of the first polyethylene glycol and is lower than or equal to body temperature.

In the present embodiment, the mixture ratio in the first agent M1 of the pine bark extract and the first polyethylene glycol is 1:4 (that is, the pine bark extract has 20 weight percent, the first polyethylene glycol 80 weight percent). The mixture ratio in the second agent M2 of the pine bark extract and the second polyethylene glycol is 1:4 (that is, the pine bark extract has 20 weight percent, the second polyethylene glycol 80 weight percent). That is, the first polyethylene glycol is a main ingredient of the first agent M1, and the second polyethylene glycol is a main ingredient of the second agent M2.

Further, the tampon body 20 according to the present embodiment, as shown in FIG. 3A, has a pattern on the outer surface 21. In other words, the tampon body 20 includes on its outer surface 21 the applied portions 23 to which the agent M is applied and non-applied portions 25 to which the agent M is not applied. Also, the pattern is formed on the outer surface 21 by the applied portions 23 and non-applied portions 25 (that is, by the difference in color between the applied portions 23 and non-applied portions 25). The pattern according to the present embodiment is one that is formed by arranging alternately regularly the applied portions 23 and non-applied portions 25. Specifically, the pattern, as shown in FIG. 3A, is one that is composed of rings (4 mm in width) lined up in the longitudinal direction of the tampon body 20.

Onto the tampon body 20 according to the present embodiment, a withdrawal string 22 as an example of a string is stitched. This withdrawal string 22 is a cotton string. The withdrawal string 22 extends from the rear end of the tampon body 20, and is held by a user of the tampon while attempting to remove the tampon body 20 out of the vaginal cavity. Further, as shown in FIGS. 1 and 2, the withdrawal string 22 passes inside the applicator 30 and extends somewhat beyond the rear end of the applicator 30 (the inner tube 50). That is, a part of the withdrawal string 22 exposes outside from the rear end of the applicator 30 (the inner tube 50).

In the present embodiment, while the agent M is applied to the tampon body 20 (the applied portions 23 are included), the agent M is not applied to an exposed portion 22a of the withdrawal string 22 (the applied portions 23 are not included). Further, the withdrawal string 22 does not include any of the applied portions 23 (There is no applied portion 23 on the withdrawal string 22).

The applicator 30 is an assisting tool in order to facilitate insertion of the tampon body 20 into the vaginal cavity. The applicator 30 includes the outer tube 40 and the inner tube 50, as shown in FIGS. 1 and 2.

The outer tube 40 is for housing the tampon body 20. The outer tube 40 is a cylinder that is injection-molded from thermoplastic resin (in the present embodiment, polyethylene resin), and has suitable flexibility. The outer tube 40 has a transparency that allows the tampon body 20 housed in the outer tube to be seen from outside (in other words, the pattern formed on the tampon body 20) (for example, haze value of 90% or less; in the present embodiment, 47%). The outer tube 40 may or may not be colored; in the present embodiment, an entire surface of the outer tube 40 is colored light pink.

Further, the outer tube 40 includes: a radially-large portion 41 positioned on the front end side (in other words, the one end side in the longitudinal direction of the outer tube 40), and a radially-small portion 42 that has an internal diameter smaller than that of the radially-large portion 41 and is positioned on the rear end side, opposite the front end side (in other words, the other end side in the longitudinal direction of the outer tube 40) (the radially-large portion 41 is also larger than the radially-small portion 42 in external diameter). The front end section of the outer tube 40 is larger than the rear end thereof in external diameter (internal diameter). Thereby, an annular shoulder 47 is formed between the radially-large portion 41 and radially-small portion 42.

The radially-large portion 41 is a portion of the outer tube 40 and has a function mainly to house the tampon body 20 inside thereof. Indeed, in the tampon 10 according to the present embodiment, the tampon body 20 is housed in the radially-large portion 41 only of the radially-large portion 41 and radially-small portion 42 (therefore, in the longitudinal direction of the outer tube 40, the length of the radially-large portion 41 is larger than that of the tampon body 20). The radially-large portion 41 is a portion that is inserted into the vaginal cavity with housing the tampon body 20 therein when the tampon 10 is used.

Further, the radially-large portion 41 (the outer tube 40) includes an opening (hereinafter referred to as a front-end opening 43) on its front end, and also includes a plurality of petal portions 44 surrounding the front-end opening 43 (in the present embodiment, 6). Each of the plurality of petal portions 44 is bent in the form of an arc radially inwardly of the outer tube 40, as shown in FIG. 4. Therefore, when inserting the outer tube 40 into the vaginal cavity, the front end section of the outer tube 40 is substantially hemispherical in shape as shown in FIGS. 1 and 2, and the front-end opening 43 is substantially closed as shown in FIG. 5. When the tampon body 20 is expelled from the front-end opening 43 by the inner tube 50 described below, the front-end opening 43 opens.

The radially-small portion 42 is a section of the outer tube 40 that provides a space in which the following inner tube 50 mainly moves (However, of course, the inner tube 50 moves not only inside the radially-small portion 42 but also inside the radially-large portion 41). The radially-small portion 42 is a portion that is held by a user when the tampon 10 is used.

Further, the radially-small portion 42 (the outer tube 40) includes an opening (hereinafter referred to as a rear-end opening 45) on the rear end as shown in FIG. 4, and also includes an annular rib 46 that is formed slightly closer to the front end than the rear-end opening 45 is.

As shown in FIGS. 1, 2, 6, and 7, the outer tube 40 includes ribs (hereinafter referred to as a longitudinal ribs 54) on an inner surface 40a thereof (of the outer tube 40) along the longitudinal direction of the outer tube 40. The outer tube 40 according to the present embodiment includes the longitudinal ribs 54 in at least an area that is closer to the front end from a center C in the longitudinal direction of the outer tube 40 (see FIG. 1). Also, in the present embodiment, the outer tube 40 includes the longitudinal ribs 54, on only the radially-large portion 41 of the radially-large portion 41 and the radially-small portion 42.

As shown in FIG. 6, 32 of the longitudinal direction ribs 54 are arranged such that the ribs are equally spaced along the inner circumferential direction of the inner surface 40a. In other words, the outer tube 40 (radially-large portion 41) has three or more longitudinal direction ribs 54 that are arranged such that the ribs 54 are equally spaced along the inner circumferential direction of the inner surface 40a. In the present embodiment, the longitudinal direction ribs 54 that are adjacent to each other are not in contact with each other (do not abut and touch each other).

Each of the longitudinal direction ribs 54 is formed straight from the front end of the radially-large portion 41 to the rear end thereof, as shown in FIGS. 1 and 2. More specifically, the longitudinal direction ribs 54 according to the present embodiment are disposed of the radially-large portion 41 up to an rearmost end E2 thereof, but do not reach an foremost end of the radially-large portion 41. In other words, the petal portions 44 do not have the longitudinal rib 54, and the ribs 54 are disposed on the radially-large portion 41 up to a foremost end E1 thereof except for the petal portions.

Further, as shown in FIG. 6, the longitudinal rib 54 according to the present embodiment is a rib extending radially of the radially-large portion 41, and the width of the rib narrows as it gets closer radially to the center. As shown in FIG. 7, the height h of the longitudinal rib 54 extending radially of the radially-large portion 41 is smaller than the difference of the internal diameters of the radially-large portion 41 and radially-small portion 42 (internal diameter of radially-large portion 41 R - internal diameter of radially-small portion r = R-r) (h < R-r). In other words, the internal diameter R-h of the radially-large portion 41 considering the longitudinal ribs 54 (hereinafter referred to as, for convenience, a rib-considered internal diameter) is larger than the internal diameter r of the radially-small portion (r < R-h) .

The tampon body 20 has an external diameter substantially the same as the rib-considered internal diameter, and the tampon body 20 is housed in the radially-large portion 41 of the outer tube 40, being in contact only with the longitudinal rib 54 of the inner surface 40a and the longitudinal rib 54, as shown in FIG. 6. In other words, the outer surface 21 of the tampon body 20 is not in contact with the inner surface 40a, and is in contact only with a radially-extending front end section of the longitudinal ribs 54.

The inner tube 50 is for expelling the tampon body 20 from the front-end opening 43 outside the outer tube 40 by moving in the outer tube 40. This inner tube 50 is inserted into the outer tube 40, and is positioned closer to the rear end in the outer tube 40 than the tampon body 20 is. The inner tube 50 moves along the longitudinal direction of the outer tube 40 and pushes the tampon body 20 towards the front-end opening 43 from the rear. Thereby, the tampon body 20 pushes aside each of the plurality of petal portions 44 radially outwardly of the outer tube 40 (in other words, opens the front-end opening 43) and is expelled from the outer tube 40. As mentioned above, the inner tube 50 has a function to expel the tampon body 20 out of the outer tube 40 by moving the outer tube 40.

Further, the inner tube 50 according to the present embodiment has a retractable configuration in order to make the tampon 10 compact in size. Specifically, as shown in FIG. 1, when the inner tube 50 is shortened, the inner tube 50 is shorter in length than the outer tube 40 so that the length of the tampon 10 is suitable for carrying. On the other hand, as shown in FIG. 2, when the inner tube 50 is elongated, the length of the inner tube 50 is sufficient to expel the tampon body 20 outside the outer tube 40. As mentioned above, in order to make the inner tube 50 retractable, in the present embodiment, the inner tube 50 has a dual structure. Specifically, as shown in FIGS. 1 and 2, the inner tube 50 of the present embodiment includes a first inner tube 51, and a second inner tube 52 that is slidably inserted into the first inner tube 51.

The first inner tube 51 is a cylinder that is injection-molded from plastic. The first inner tube 51 has an external diameter that is slightly smaller than the internal diameter of the radially-small portion 42 of the outer tube 40. Also, the first inner tube 51 is slidably inserted into the radially-small portion 42, as shown in FIG. 1. On the outer circumferential face of the front end section of the first inner tube 51, an annular sword-guard portion 51a is formed. This sword-guard portion 51a has an external diameter that is slightly smaller than the rib-considered internal diameter of the radially-large portion 41 of the outer tube 40. Also, the sword-guard portion 51a is stopped by connecting it to an inner wall of the shoulder 47 so that the sword-guard portion 51a prevents the inner tube 50 from falling off the rear-end opening 45 of the outer tube 40. When the inner tube 50 expels the tampon body 20 out of the outer tube 40, the inner tube 50 moves such that the outer circumferential face of the sword-guard portion 51a comes into contact with the longitudinal ribs 54 of the radially-large portion 41. Further, at the rear end section of an inner circumferential face of the first inner tube 51, an annular projection 51b extending radially inwardly of the first inner tube 51 is formed, as shown in FIGS. 1 and 2.

The second inner tube 52 is a cylinder that is injection-molded from thermoplastic resin. This second inner tube 52 has an external diameter slightly smaller than the internal diameter of the first inner tube 51. The second inner tube 52 is inserted into the first inner tube 51 as shown in FIG. 1 when the inner tube 50 is shortened. The second inner tube 52 is connected to the rear end section of the first inner tube 51 at the front end section of the second inner tube 52 as shown in FIG. 2 when the inner tube 50 is elongated. On the outer circumferential face of the front end section of the second inner tube 52, are formed an arc-shaped sword-guard portion 52a and a projection section 52b that is located closer to the rear end than the sword-guard portion 52a is. The height of the projection section 52b is lower as it gets close to the rear end, as shown in FIG. 2. The space between the sword-guard portion 52a and projection section 52b of the second inner tube 52 is slightly larger than the thickness of the annular projection 51b of the first inner tube 51.

When the second inner tube 52 is pulled towards the rear end, the annular projection 51b of the first inner tube 51 is positioned between the sword-guard portion 52a and projection section 52b of the second inner tube 52. In this state, as shown in FIG. 2, the annular projection 51b is stopped by connecting to the sword-guard portion 52a and projection section 52b, and the first inner tube 51 connects to the second inner tube 52.

Further, as shown in FIGS. 1 and 2, a flared portion 52c is formed on rear end section of the second inner tube 52. The external diameter of the flared portion 52c is desirably at least larger than the internal diameter of the first inner tube 51 and larger than or equal to the internal diameter of the radially-small portion 42 of the outer tube 40.

### === Effectiveness of Tampon 10 according to Present Embodiment ===

As mentioned above, in the tampon 10 according to the present embodiment, the tampon body 20 includes on the outer surface 21 the applied portion 23 to which the agent M is applied. The applied portion 23 includes at least two applied layers. The at least two applied layers are: the first applied layer 24a formed by applying the first agent M1; and the second applied layer 24b formed by applying the second agent M2 over the first applied layer 24a, the first agent M1 having the pine bark extract as an example of an active pharmaceutical ingredient and the first polyethylene glycol as an example of a first water-soluble carrier that carries the pine bark extract, the second agent M2 having the pine bark extract and the second polyethylene glycol as an example of a second water-soluble carrier that carries the pine bark extract and whose melting point is lower than that of the first polyethylene glycol. Thereby, the tampon 10 having the agents M that properly take effect while the tampon is inserted in the vaginal cavity is achieved.

Regarding the foregoing, the description will be made comparing the tampon 10 according to the present embodiment (the present example) and a tampon according to comparative example. Comparing the tampon 10 according to the present embodiment to the tampon according to comparative example, the tampon body 20 in both of them includes on its outer surface 21 the applied portions 23 to which the agent M is applied, and also that the agent M is the mixture of the pine bark extract and the polyethylene glycol. However, in the tampon according to comparative example, the applied layer is a single layer, and in the applied layer one type of polyethylene glycol alone is mixed with the pine bark extract.

Consider the case where a polyethylene glycol having a low melting point (for example, the foregoing second polyethylene glycol whose melting point is lower than or equal to body temperature) is selected. When storing a tampon in a place where the temperature is high (a warehouse, for example), the agent melts. This may cause a problem that the melted agent is absorbed into or falls off the tampon body. When using the tampon in which the foregoing absorption or falling off has happened (that is, inserting the tampon into the vaginal cavity), the amount of the agent adhering onto the outer surface decreases. Therefore, the agent cannot be transferred to the vaginal mucosa appropriately. That is, the agent does not properly take effect when the tampon is inserted into the vaginal cavity.

On the other hand, in the case where a polyethylene glycol having high melting point (for example, the foregoing first polyethylene glycol whose melting point is higher than body temperature) is selected, the agent is less likely to melt even when the tampon is inserted into the vaginal cavity. Melting of the agent is considerably delayed (melting of the agent is delayed until menstruation occurs, that is, until water such as menstrual blood etc comes out sufficiently for the polyethylene glycol to dissolve in the water; melting of the agent is less likely to happen before menstruation). In other words, the agent does not properly take effect when a tampon is inserted into the vaginal cavity (this makes the agent less rapid-acting).

As mentioned above, in the tampon according to the comparative example, regardless of the melting points, a problem that the agent does not properly take effect when a tampon is inserted into the vaginal cavity may arise.

As opposed thereto, in the present example, each applied portion 23 has at least two applied layers. This makes it possible to apply two types of agents (the first agent M1 and the second agent M2) respectively to two applied layers (the first applied layer 24a and the second applied layer 24b). Also, the polyethylene glycols having two melting points can be contained separately in the two types of the agents. That is, as the present embodiment, the first polyethylene glycol having a higher melting point than body temperature and the second polyethylene glycol having a melting point lower than or equal to body temperature can be contained separately in the first agent M1 and the second agent M2. Further, the foregoing problems are properly solved by placing the second applied layer 24b over the first applied layer 24a, the second applied layer 24b including the second polyethylene glycol having a lower melting point, the first applied layer 24a including the first polyethylene glycol having a higher melting point.

That is, even if storing the tampon 10 in a place where the temperature is high (a warehouse, for example) results in melting of the second polyethylene glycol having a low melting point (the second agent M2), the frozen first polyethylene glycol (the first agent M1) in the first applied layer 24a located inside properly prevents the melted second polyethylene glycol (the second agent M2) in the second applied layer 24b located outside from being absorbed into the tampon body. This prevents the foregoing problems of absorption. Also, even if melting of the second polyethylene glycol (the second agent M2) causes the second polyethylene glycol of the second applied layer 24b (the second agent M2) to fall off, the first polyethylene glycol of the first applied layer 24a (the first agent M1) remains. This can make the degree of falling off less than with the comparative example in which one type of the polyethylene glycol is used and it falls off. This makes it possible to properly avoid the foregoing problem that the agent M cannot be transferred properly to the vaginal mucosa due to decreasing of the amount of the agent M adhering onto the outer surface 21.

Further, when inserting the tampon 10 into the vaginal cavity, the first polyethylene glycol (the first agent M1) is frozen, but the second polyethylene glycol (the second agent M2) is melted because of body temperature. Therefore, the second polyethylene glycol (the second agent M2) of the first applied layer 24a located outside is immediately transferred to the vaginal mucosa. That is, even before water such as menstrual blood etc comes out sufficiently (for example, before menstruation), the agent M properly takes effect (the agent M can be more rapid-acting) because of action of the second agent M2. In such a state as menstruation in which water such as menstrual blood etc comes out sufficiently and the first polyethylene glycol (the first agent M1) dissolves in the water, the agent M properly takes effect because of action of the first agent M1. That is, this enables the agent M to take effect gradually and ideally.

As mentioned above, according to the present embodiment, the tampon 10 having the agent M that properly takes effect while the tampon is inserted in the vaginal cavity is achieved.

Further, the second applied layer 24b according to the present embodiment is a layer formed by applying the second agent M2 over the first applied layer 24a such that the second applied layer 24b does not exceed the boundaries of the first applied layer 24a. Therefore, if storing the tampon 10 in a place where the temperature is high (a warehouse, for example) results in melting of the second agent M2, the first agent M1 of the first applied layer 24a located inside prevents more definitely the second agent M2 of the second applied layer 24b located outside from being absorbed into the tampon body. Therefore, the tampon 10 having the agent M that more properly takes effect while the tampon is inserted in the vaginal cavity is achieved.

Further, in the present embodiment, on the outer surface 21 of the tampon body 20, the tampon body 20 includes the applied portion 23 to which the agent M is applied and the non-applied portion 25 to which the agent M is not applied. Also, by the applied portions 23 and the non-applied portions 25, the pattern is formed on the outer surface 21. This makes the appearance of the tampon body 20 good, and can make the tampon 10 appear attractive (this effect leads to less reluctance to insert the tampon 10). Further, the applied portion 23 has at least two applied layers. The two applied layers are: the first applied layer 24a formed by applying the first agent M1; and the second applied layer 24b formed by applying the second agent M2 over the first applied layer 24a, the first agent M1 having the pine bark extract as an example of active pharmaceutical ingredient and the first polyethylene glycol as an example of the first water-soluble carrier that carries the pine bark extract, the second agent M2 having the pine bark extract and the second polyethylene glycol as an example of the second water-soluble carrier that carries the pine bark extract and whose melting point is lower than that of the first polyethylene glycol. As mentioned above, if the agent M falls off the tampon body 20 while the tampon 10 is stored, this can make the degree of falling off less (even if the second agent M2 falls off, the first agent M1 remains). Therefore, in the tampon 10 according to the present embodiment, the pattern is less likely to distort and it can definitely make the tampon 10 appear attractive.

Further, in the foregoing embodiment, the first water-soluble carrier (the first polyethylene glycol) has a higher melting point than body temperature, and the second water-soluble carrier (the second polyethylene glycol) has a melting point lower than and equal to body temperature. Therefore, the tampon 10 having the agent M that properly takes effect while the tampon is inserted in the vaginal cavity is achieved more definitely.

### === Manufacturing Method for Tampon 10 ===

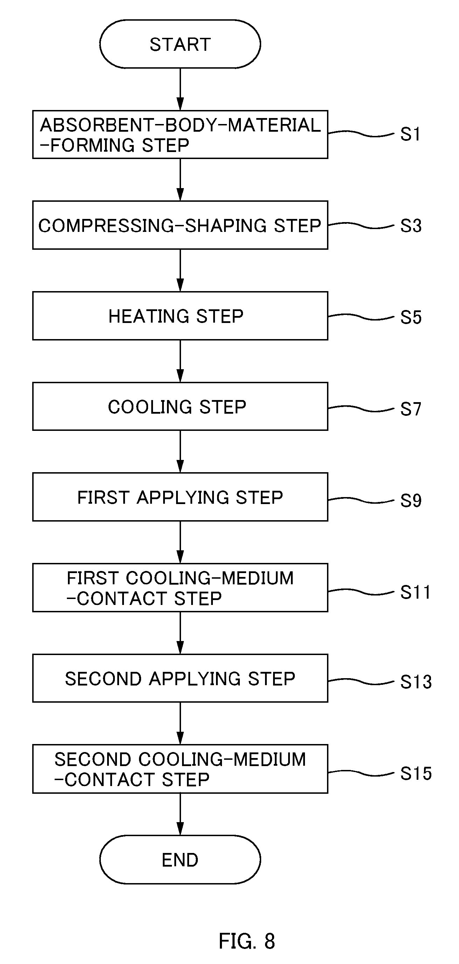

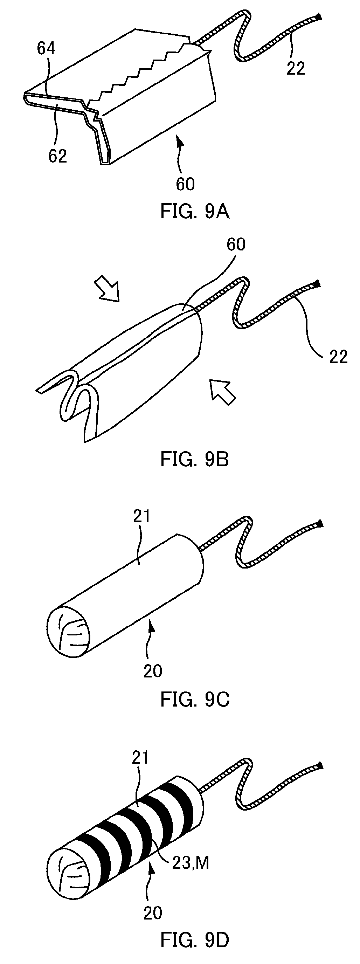

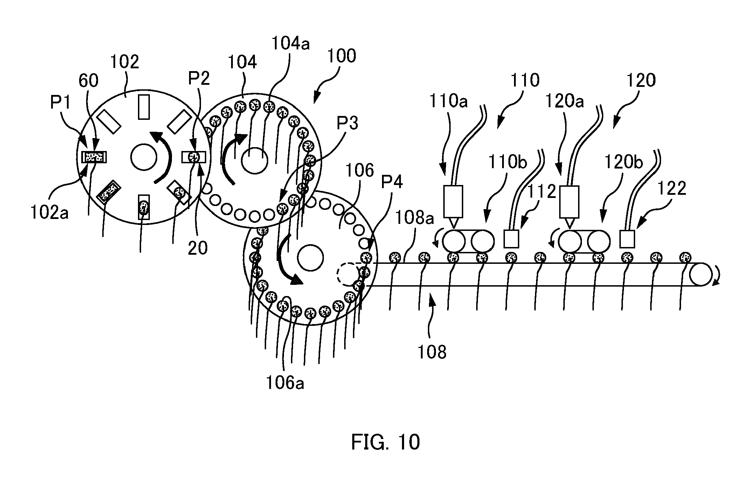

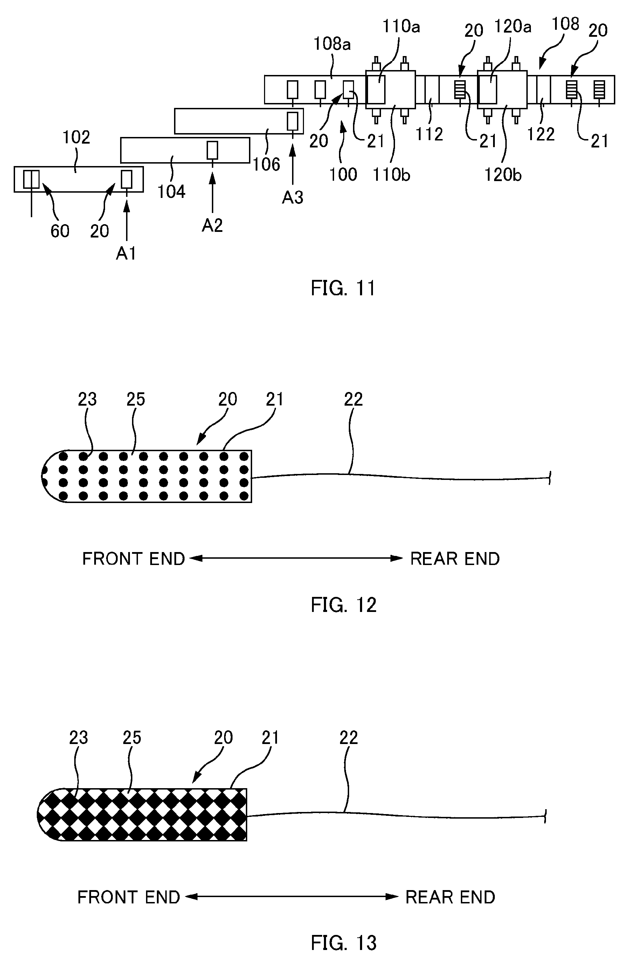

Next, a manufacturing method for manufacturing the foregoing tampon 10 will be described with reference to FIGS. 8 to 11. FIG. 8 is a flowchart showing the production flow of the tampon body 20. FIGS. 9A to 9D are schematic diagrams showing the transition of the tampon body 20 to a finished product. FIG. 10 is a schematic diagram showing a section of the manufacturing apparatus 100 for the tampon 10, which manufactures the tampon body 20. FIG. 11 is a schematic diagram of the manufacturing apparatus 100 shown in FIG. 10 viewed from above.

The manufacturing process of the tampon 10 is divided into: a process in which the components of the tampon 10 (that is, the tampon body 20, the outer tube 40, the first inner tube 51, and the second inner tube 52) are manufactured, and a process in which these components are assembled. This section will describe the process in which the tampon body 20 (more precisely, the tampon body 20 having the withdrawal string 22) is manufactured.

The production flow of FIG. 8 starts with an absorbent-body-material-forming step (step S1). In this step, firstly, the absorbent main body 62 (wool-like body) is covered with a cover 64 (wrapped with the cover 64). Then, the absorbent main body 62 covered with the cover 64 is cut into a predetermined shape and size. Thereby, an absorbent-body material 60 is formed (that is, the base material of the tampon body 20). in this step, the absorbent-body material 60 undergoes a process in which the withdrawal string 22 is stitched to the absorbent-body material 60 (FIG. 9A shows a state of the absorbent-body material 60 after the step is finished).

Next, the tampon body 20 is obtained by compressing and shaping the absorbent-body material 60 (compressing-shaping step of step S3).

FIGS. 10 and 11 show a compressing-shaping drum 102 as an example of a compressing-shaping unit; the compressing-shaping drum 102 has a function to compress and shape the absorbent-body material 60 (also, the tampon body 20 is obtained thereby). Indeed, the compressing-shaping drum 102 is a drum-shaped rotatable unit, and includes a plurality of holding sections 102a (in the present embodiment, 8) positioned radially. The absorbent-body material 60 is inserted successively into the holding section 102a at a first position P1 (FIG. 10), and the inserted absorbent-body material 60 rotates and moves to a second position P2 (FIG. 10) in conjunction with rotation of the compressing-shaping drum 102. Then, while rotating and moving, the absorbent-body material 60 is compressed from both sides thereof in the holding section 102a (FIG. 9B shows a state of the absorbent-body material 60 being compressed).

Next, by heating the tampon body 20 that is obtained by compressing and shaping the absorbent-body material 60 with the compressing-shaping drum 102, the shape of the tampon body 20 is fixed (heating step of step S5).

FIGS. 10 and 11 show a heating drum 104 as an example of a heating unit; the heating drum 104 has a function to heat the tampon body 20 that is obtained by compressing and shaping the absorbent-body material 60 with the compressing-shaping drum 102 (further, thereby the shape of the tampon body 20 is fixed). Indeed, the heating drum 104 is a drum-shaped rotatable unit whose temperature is controlled at 110 degrees, for example (a temperature between 100 and 180 degrees is preferable). The drum 104 includes multiple holding sections 104a radially positioned. The tampon body 20 is transferred successively at a second position P2 (FIG. 10) from the holding section 102a of the compressing-shaping drum 102 to the holding section 104a of the heating drum 104, by the pushing of a pusher (not shown) (the direction in which the pusher pushes the body is shown with an arrow A1 in FIG. 11). Then, the tampon body 20 that is transferred to the holding section 104a rotates and moves to the third position P3 (FIG. 10) in conjunction with rotation of the heating drum 104. While rotating and moving, the tampon body 20 is heated in the holding section 104a and the shape of the tampon body 20 is fixed. The holding section 104a is a hole having a shape corresponding to the shape of the tampon body 20 (the tampon body 20 fits in the hole). Also, the heat of the heating drum 104 is effectively conducted to the tampon body 20. Further, at the same time when transferring the tampon body 20 from the compressing-shaping drum 102 to the heating drum 104, the tampon body 20 undergoes a process in which the front end is formed in the shape of a bullet. FIG. 9C shows a state of the tampon body 20 after the heating step is finished.

Next, the tampon body 20 whose shape is fixed by the heating drum 104 is cooled (cooling step of step S7).

FIGS. 10 and 11 show a cooling drum 106 as an example of a cooling unit; the cooling drum 106 has a function to cool the tampon body 20 whose shape is fixed by the heating drum 104. Indeed, the cooling drum 106 is a drum-shaped rotatable unit whose temperature is controlled at 25°C for example, and includes multiple holding sections 106a positioned radially. The tampon body 20 is transferred successively at a third position P3 (FIG. 10) from the holding section 104a of the heating drum 104 to the holding section 106a of the cooling drum 106 by pushing of a pusher (not shown) (the direction in which the pusher pushes the body is shown with an arrow A2 in FIG. 11). Then, the tampon body 20 that is transferred to the holding section 106a rotates and moves to a fourth position P4 (FIG. 10) in conjunction with rotation of the cooling drum 106. While rotating and moving, the tampon body 20 is cooled in the holding section 106a. In similarity to the holding section 104a, the holding section 106a is a hole having a shape corresponding to the tampon body 20 (the tampon body 20 fits in the hole), and is configured such that the tampon body 20 is cooled effectively by the cooling drum 106.

The tampon body 20 that has rotated and moved to the fourth position P4 (FIG. 10) is transferred successively at the fourth position P4 from the holding section 106a of the cooling drum 106 to a conveyor unit 108 (specifically, a conveyor belt 108a disposed of the conveyor unit 108) by pushing of a pusher (not shown) (the direction in which the pusher pushes the body is shown with an arrow A3 in FIG. 11). The conveyor belt 108a is an endless conveyor belt for the tampon body; the conveyor belt 108a holds the tampon body 20 such that the longitudinal direction of the tampon body 20 is aligned in the width direction of the conveyor belt 108a. The conveyor belt 108a conveys the tampon body 20 by rotating and moving. The temperature of the conveyor belt 108a is controlled, for example, at 25°C, the conveyor belt 108a also has a function as a cooling unit that cools the tampon body 20. Indeed, the tampon body 20 whose shape is fixed by the heating drum 104 is cooled by the cooling units, first in the cooling drum 106 and second in the conveyor belt 108a,.

Next, the melted first agent M1 is applied onto the outer surface 21 of the tampon body 20, the first agent M1 having the pine bark extract and the first polyethylene glycol that carries the pine bark extract (first applying step of step S9). This first applying step is a step in which the first applied layer 24a is formed.

FIGS. 10 and 11 show a first applying unit 110 as an applying unit, and the first applying unit 110 has a function to apply melted first agent M1 onto the outer surface 21 of the tampon body 20. The first applying unit 110 includes a first supplying unit 110a and a first transferring belt 110b.

The first supplying unit 110a is for supplying the first transferring belt 110b with the melted first agent M1. In the present embodiment, this first supplying unit 110a melts the first agent M1 and applies the melted first agent M1 to the first transferring belt 110b.

The first transferring belt 110b is for transferring and applying the melted first agent M1 to the outer surface 21 while the belt is in contact with the outer surface 21 of the tampon body 20. In the present embodiment, this first transferring belt 110b is an endless conveyor belt for an agent, and conveys the first agent M1 by rotating and moving with holding the first agent M1 applied by the first supplying unit 110a. Then, the conveyed first agent M1 reaches a contact position at which the first transferring belt 110b comes into contact with the outer surface 21 of the tampon body 20 being conveyed by the conveyor belt 108a, and the first agent M1 is transferred and applied to the outer surface 21.

As shown in FIG. 10, when the first agent M1 is applied to the outer surface 21, the tampon body 20 is sandwiched between the first transferring belt 110b and the conveyor belt 108a. In the present embodiment, the speeds in the direction from left to right in FIG. 10 (hereinafter referred to as merely a left-to-right direction) are controlled such that the speed of the first transferring belt 110b is greater than that of the conveyor belt 108a. Therefore, while the tampon body 20 being sandwiched between the first transferring belt 110b and the conveyor belt 108a, the tampon body 20 rotates on the conveyor belt 108a and moves in the left-to-right direction. Therefore (because of the rotation), the first agent M1 is applied on the entire outer surface 21 of the tampon body 20 circumference-wise.

As mentioned above, the tampon body 20 is cooled by the cooling units whose temperature is controlled at 25°C, first by the cooling drum 106 and second by the conveyor belt 108a. Therefore, the first applying unit 110 applies the melted first agent M1 onto the outer surface 21 of the tampon body 20, the outer surface 21 being cooled to approximately 25°C by the cooling units. Further, because the melting point (freezing point) of the first water-soluble carrier of the first agent M1 (the first polyethylene glycol) is approximately 45°C as mentioned above, the melted first agent M1 freezes rapidly (instantly) when the first agent M1 is applied onto the outer surface 21 of the tampon body 20. As mentioned above, in the cooling step of step S7, the cooling units cool the tampon body 20 whose shape is fixed by the heating drum 104, such that a temperature of the outer surface 21 becomes a temperature lower than or equal to the freezing point of the first water-soluble carrier of the first agent M1 (the first polyethylene glycol). Also, in the first applying step of step S9, the first applying unit 110 applies the melted first agent M1 onto the outer surface 21 of the tampon body 20, the outer surface 21 having a temperature lower than or equal to the freezing point of the first water-soluble carrier (the first polyethylene glycol) (that is, being cooled to a temperature lower than or equal to the freezing point of the first water-soluble carrier (the first polyethylene glycol)).

Next, a cooling medium is brought into contact with the first agent M1 which is applied to the outer surface 21, the cooling medium being cooled to a temperature lower than or equal to the freezing point of the first water-soluble carrier of the first agent M1 (the first polyethylene glycol) (in the present embodiment, approximately 45°C) (the first cooling-medium-contact step of step S11).

FIGS. 10 and 11 show a first cool-air-blowing unit 112 as a first cooling-medium-contact unit that brings the cooling medium into contact; the first cool-air-blowing unit 112 has a function to bring cool air into contact with the first agent M1 that is applied to the outer surface 21 by the first applying unit 110, the cool air having a function as a cooling medium that is cooled to a temperature lower than or equal to the freezing point of the first water-soluble carrier of the first agent M1 (first polyethylene glycol). That is, the first cool-air-blowing unit 112 brings the cooling medium into contact with the first agent M1 by blowing cool air onto the first agent M1, the cool air being cooled to a temperature lower than or equal to 45°C (in the present embodiment, 25°C).

Further, as mentioned above, the temperature of the conveyor belt 108a is controlled at 25°C, the conveyor belt 108a is cooled, and further, the tampon body 20 rotates on the conveyor belt 108a with being sandwiched between the first transferring belt 110b and the conveyor belt 108a. Therefore, the first agent M1 which is applied to the outer surface 21 is brought into contact with the conveyor belt 108a instantly. Therefore, the conveyor belt 108a also has a function as a cooling medium that is cooled to a temperature lower than or equal to the freezing point of the first water-soluble carrier (first polyethylene glycol). In other words, the conveyor unit 108 has a function as a first cooling-medium-contact unit that brings the conveyor belt 108a into contact with the first agent M1 applied to the outer surface 21, the conveyor belt 108a having a function as a cooling medium that is cooled to a temperature lower than or equal to the freezing point of the first water-soluble carrier (the first polyethylene glycol).

As mentioned above, in the present embodiment, the melted first agent M1 is applied onto the outer surface 21 of the tampon body 20, the outer surface 21 being cooled to a temperature lower than or equal to the freezing point of the first water-soluble carrier (the first polyethylene glycol). In addition thereto, the cooling medium is brought into contact with the first agent M1 applied to the outer surface 21, the cooling medium being cooled to a temperature lower than or equal to the freezing point to the first water-soluble carrier (the first polyethylene glycol). Therefore, when the melted first agent M1 is applied to the outer surface 21 of the tampon body 20, the first agent M1 freezes more rapidly.

Next, the melted second agent M2 is applied over the first agent M1 (the second applying step of step S13), the second agent M2 having the pine bark extract and the second polyethylene glycol that carries the pine bark extract and whose melting point is lower than that of the first polyethylene glycol. The second applying step is a step in which the second applied layer 24b is formed.

FIGS. 10 and 11 show a second applying unit 120 as an applying unit; the second applying unit 120 has a function to apply the melted second agent M2 over the first agent M1. The second applying unit 120 includes a second supplying unit 120a and a second transferring belt 120b.

The second supplying unit 120a and the second transferring belt 120b have the same configuration as the foregoing first supplying unit 110a and first transferring belt 110b.

The second supplying unit 120a is for supplying the second transferring belt 120b with the melted second agent M2. In the present embodiment, this second supplying unit 120a melts the second agent M2 and applies the melted second agent M2 to the second transferring belt 120b.

The second transferring belt 120b is for transferring and applying the melted second agent M2 to the outer surface 21 (the first applied layer 24a) while the belt is in contact with the outer surface 21 of the tampon body 20 (specifically, the first applied layer 24a on the outer surface 21). In the present embodiment, this second transferring belt 120b is an endless conveyor belt for an agent. The second transferring belt 120b conveys the second agent M2 that is applied with the second supplying unit 120a, by rotating and moving with holding the second agent M2. Then, the conveyed second agent M2 reaches a contact position at which the second transferring belt 120b comes into contact with the outer surface 21 (the first applied layer 24a) of the tampon body 20 being conveyed by the conveyor belt 108a, the second agent M2 is transferred and applied to the first applied layer 24a. That is, the second agent M2 is applied over the first agent M1.

As shown in FIG. 10, when the second agent M2 is applied over the first agent M1, the tampon body 20 is sandwiched between the second transferring belt 120b and the conveyor belt 108a. In the present embodiment, the speeds in the direction from left to right in FIG. 10 (hereinafter referred to as merely a left-to-right direction) are controlled such that the speed of the second transferring belt 120b is greater than that of the conveyor belt 108a. Therefore, the tampon body 20 rotates on the conveyor belt 108a and moves in the left-to-right direction with being sandwiched between the second transferring belt 120b and the conveyor belt 108a. Therefore (because of the rotation), the second agent M2 is applied onto the entire outer surface 21 of the tampon body 20 circumference-wise.

As mentioned above, in the first cooling-medium-contact step of step S11, the cooling medium (cool air and conveyor belt 108a) that is cooled to 25°C comes into contact with the first agent M1. Therefore, the second applying unit 120 applies the melted second agent M2 over the first agent M1 that is cooled to approximately 25°C. Further, because the melting point (freezing point) of the second water-soluble carrier of the second agent M2 (the second polyethylene glycol) is approximately 37°C as mentioned above, the melted second agent M2 freezes rapidly (instantly) when the second agent M2 is applied over the first agent M1. As mentioned above, in the first cooling-medium-contact step of step S11, the first cooling-medium-contact unit cools the first agent M1 to a temperature lower than or equal to the freezing point of the second water-soluble carrier of the second agent M2 (the second polyethylene glycol). Also, in the second applying step of step S13, the second applying unit 120 applies the melted second agent M2 over the first agent M1, the first agent M1 having a temperature lower than or equal to the freezing point of the second water-soluble carrier (the second polyethylene glycol) (that is, being cooled to a temperature lower than or equal to the freezing point of the second water-soluble carrier (the second polyethylene glycol)).

Next, a cooling medium is brought into contact with the second agent M2, the cooling medium cooled to a temperature lower than or equal to the freezing point of the second water-soluble carrier of the second agent M2 (the second polyethylene glycol) (in the present embodiment, approximately 37°C) (the second cooling-medium-contact step of step S15).

FIGS. 10 and 11 show a second cool-air-blowing unit 122 as a second cooling-medium-contact unit that brings the cooling medium into contact; the second cool-air-blowing unit 122 has a function to bring cool air into contact with the second agent M2 which is applied over the first agent M1 by the second applying unit 120, the cool air having a function as a cooling medium that is cooled to a temperature lower than or equal to the freezing point of the second water-soluble carrier of the second agent M2 (the second polyethylene glycol). That is, the second cool-air-blowing unit 122 brings the cooling medium into contact with the second agent M2 by blowing cool air onto the second agent M2, the cool air being cooled to a temperature lower than or equal to 37°C (in the present embodiment, 25°C).

Further, as mentioned above, the temperature of the conveyor belt 108a is controlled at 25°C, the conveyor belt 108a is cooled, and further the tampon body 20 rotates on the conveyor belt 108a with being sandwiched between the second transferring belt 120b and the conveyor belt 108a. Therefore, the second agent M2 comes instantly into contact with the conveyor belt 108a. Therefore, the conveyor belt 108a also has a function as a cooling medium that is cooled to a temperature lower than or equal to the freezing point of the second water-soluble carrier (the second polyethylene glycol). In other words, the conveyor unit 108 has a function as a second cooling-medium-contact unit that brings the conveyor belt 108a into contact with the second agent M2 applied over the first agent M1, the conveyor belt 108a having a function as a cooling medium that is cooled to a temperature lower than or equal to the freezing point of the second water-soluble carrier (the second polyethylene glycol).

As mentioned above, in the present embodiment, the melted second agent M2 is applied over the first agent M1 that is cooled to a temperature lower than or equal to the freezing point of the second water-soluble carrier (the second polyethylene glycol). In addition thereto, the cooling medium comes into contact with the applied second agent M2, the cooling medium being cooled to a temperature lower than or equal to the freezing point of the second water-soluble carrier (the second polyethylene glycol). Therefore, the melted second agent M2 is applied over the first agent M1, the second agent M2 freezes more rapidly. The second cooling-medium-contact step is performed, and the manufacturing process of the tampon body 20 is finished. FIG. 9D shows a state of the tampon body 20 after the second cooling-medium-contact step is finished.

As stated above, the manufacturing method (manufacturing apparatus 100) for the tampon 10 according to the present embodiment includes: compressing-shaping step (compressing-shaping drum 102) in which the absorbent-body material 60 is obtained by compressing and shaping the tampon body 20; the first applying step (applying unit) in which the melted first agent M1 is applied onto the outer surface 21 of the tampon body 20, the first agent M1 having the pine bark extract and the first polyethylene glycol that carries the pine bark extract, the outer surface 21 having a temperature lower than or equal to the freezing point of the first polyethylene glycol; and the second applying step (applying unit) in which the melted second agent M2 is applied over the first agent M1, the second agent M2 having the pine bark extract and the second polyethylene glycol that carries the pine bark extract and whose melting point is lower than that of the first polyethylene glycol, the first agent M1 having a temperature lower than or equal to the freezing point of the second polyethylene glycol. Thereby, the manufacturing method (manufacturing apparatus 100) for the tampon 10 having agents M (the first agent M1 and the second agent M2) that properly take effect while the tampon is inserted in the vaginal cavity is achieved.

Regarding the foregoing, the description will be made comparing the manufacturing method (manufacturing apparatus 100) for the tampon 10 according to the present embodiment (the present example) and two manufacturing methods (manufacturing apparatus) for a tampon according to comparative example.

The first comparative example is a manufacturing method (manufacturing apparatus) in which the first agent and the second agent (hereinafter referred to merely as the agents) are applied onto the outer surface of the tampon body prior to compressing and shaping the absorbent-body material. For example, in the comparative example, after the agents are applied onto a cover, the absorbent main body is covered with the cover and the absorbent-body material is formed. Thereafter, the formed absorbent-body material is compressed and shaped to obtain the tampon body.

However, such a manufacturing method (manufacturing apparatus) may cause the following problems. Indeed, because the agents are applied onto the absorbent-body material prior to compressing and shaping, the agents may fall off when the absorbent-body material is compressed and shaped. In the case of using such a tampon as the foregoing when falling off has happened (in other words, in the case of inserting such a tampon into the vaginal cavity), the agents cannot be transferred to the vaginal mucosa properly because the amount of the agents adhering onto the outer surface decreases. That is, the agent does not properly take effect when a tampon is inserted into vaginal cavity. Also, the falling off may cause a problem that the manufacturing apparatus becomes soiled with the agent.

Further, in order to avoid this problem, a second comparative example is a manufacturing method (manufacturing apparatus) in which the tampon body is dipped into the agents after obtaining the tampon body by compressing and shaping the absorbent-body material. However, in such a manufacturing method (manufacturing apparatus), when the tampon body is dipped into the agents, the agents may be absorbed into the tampon body. In the case of using such a tampon as the foregoing when absorption has happened (in other words, in the case of inserting such a tampon into the vaginal cavity), the amount of the agent adhering onto the outer surface decreases. Therefore, the agent cannot be transferred to the vaginal mucosa appropriately. As a result, that is, the agent does not properly take effect when a tampon is inserted into the vaginal cavity. Further, the absorption may cause a problem that the tampon body becomes too large in size.

As mentioned above, in both of manufacturing methods (manufacturing apparatuses) for a tampon according to the comparative examples, the problem that the agents do not properly take effect when the manufactured tampon is inserted into the vaginal cavity may arise.

As opposed thereto, in the manufacturing method (manufacturing apparatus 100) for the tampon 10 according to the present embodiment, after obtaining the tampon body 20 by compressing and shaping the absorbent-body material 60, the agent M is applied to the outer surface 21 of the tampon body 20. Therefore, the falling-off problem will not arise. Also, the melted first agent M1 is applied to the outer surface 21 of the tampon body 20 having a temperature lower than or equal to the freezing point of the first polyethylene glycol. The melted second agent M2 is applied over the first agent M1 having a temperature lower than or equal to the freezing point of the second polyethylene glycol. Therefore, when the melted first agent M1 and second agent M2 is applied, the first agent M1 and second agent M2 freeze rapidly (instantly) on the outer surface 21. As a result, the first agent M1 and second agent M2 are not absorbed deeper than the outer surface 21 (in other words, inside of the tampon body).

As mentioned above, in the present embodiment, the falling off or absorption is prevented. Therefore, the foregoing problem that the agent M cannot be transferred appropriately to the vaginal mucosa due to decreasing the amount of the agent M adhering onto the outer surface 21 is avoided properly. That is, the manufacturing method (manufacturing apparatus 100) for the tampon 10 having the agent M that properly takes effect while the tampon is inserted in the vaginal cavity is achieved. Also, in the present embodiment, the problem of the manufacturing apparatus soiled with the agent caused by falling off or the problem of the oversized tampon body caused by absorption is avoided properly. Further, in the present embodiment, the falling off or absorption is prevented. This allows a pattern to be formed nicely on the outer surface 21.

### ===Other Embodiments ===

Above, based on the above embodiments, the tampon according to the invention is described, but the above embodiments of the invention are for facilitating understanding of the invention, and are not limiting of the invention. The invention can of course be altered and improved without departing from the gist thereof, and equivalents are intended to be embraced therein.

Further, in the foregoing embodiment, the tampon 10 having the applicator 30 is provided as an example of a tampon, but the invention is not limited thereto. A tampon without an applicator can be employed.

Further, in the foregoing embodiment, a pattern that is composed of rings lined up in the longitudinal direction of the tampon body 20 is provided as an example of a pattern, but the invention is not limited thereto. For example, a spotted pattern shown in FIG. 12 or a checkerboard pattern shown in FIG. 13 also can be employed. FIGS. 12 and 13 correspond to FIG. 3A and are diagrams showing a pattern according to the other embodiment.

Further, in the foregoing embodiment, the pine bark extract is provided as an example of an active pharmaceutical ingredient of the agent M, but the invention is not limited thereto. For example, a plant extract such as red clover, polygonum indigo extract, indirubin or the like can be employed. In addition thereto, flavangenol ® is provided as an example of a pine bark extract, but the invention is not limited thereto. For example, pycnogenol ® which Nihon SiberHegner K.K deals in or enzogenol ® which Valentine Company Limited deals in can be employed.

Further, in the foregoing embodiment, polyethylene glycol with a molecular weight of 1000 (with melting point at approximately 37°C) is provided as an example of the second water-soluble carrier, but the invention is not limited thereto. For example, polyethylene glycol with a molecular weight of 600 (with melting point at approximately 20°C) can be employed.

The polyethylene glycol with molecular weight of 1000 is difficult to melt when the tampon 10 is stored, and is superior because this makes it possible to properly prevent the polyethylene glycol from absorption or falling off as mentioned above. The polyethylene glycol with molecular weight of 600 is easy to melt when inserting the tampon 10 into the vaginal cavity, which makes the agent M more rapid-acting. In this point, the polyethylene glycol with molecular weight of 600 is superior.

### Reference Signs List

10 tampon, 20 tampon body (absorbent body),
21 outer surface, 22 withdrawal string, 22a exposed portion,
23 applied portion, 24a first applied layer, 24b second applied layer,
25 non-applied portion, 30 applicator, 40 outer tube,
40a inner surface, 41 radially-large portion, 42 radially-small portion, 43 front-end opening,
44 petal portion, 45 rear-end opening, 46 annular rib, 47 shoulder,
50 inner tube, 51 first inner tube, 51a sword-guard portion, 51b annular projection,
52 second inner tube, 52a sword-guard portion, 52b projection section, 52c flared portion,
54 longitudinal rib, 60 absorbent-body material, 62 absorbent main body, 64 cover,
100 manufacturing apparatus, 102 compressing-shaping drum, 102a holding section,
104 heating drum, 104a holding section, 106 cooling drum,
106a holding section, 108 conveyor unit, 108a conveyor belt,
110 first applying unit (applying unit), 110a first supplying unit,
110b first transferring belt, 112 first cool-air-blowing unit,
120 second applying unit (applying unit), 120a second supplying unit,
120b second transferring belt, 122 second cool-air-blowing unit,
C center, E1 foremost end, E2 rearmost end,
M agent, M1 first agent, M2 second agent

## Claims

1. A tampon having an absorbent body that absorbs liquid, comprising:
on an outer surface of the absorbent body,
an applied portion to which an agent is applied and that includes at least two applied layers having
a first applied layer formed by applying a first agent, and
a second applied layer formed by applying a second agent over the first applied layer,
the first agent including an active pharmaceutical ingredient and a first water-soluble carrier that carries the active pharmaceutical ingredient,
the second agent including an active pharmaceutical ingredient and a second water-soluble carrier that carries the active pharmaceutical ingredient and has a melting point lower than that of the first water-soluble carrier.

2. A tampon according to claim 1, wherein
the second applied layer is a layer formed by applying the second agent over the first applied layer such that the second applied layer does not exceed a boundary of the first applied layer.

3. A tampon according to claim 1 or 2, wherein
the absorbent body includes, on the outer surface thereof, the applied portion to which the agent is applied and a non-applied portion to which the agent is not applied, and
a pattern is formed by the applied portion and the non-applied portion on the outer surface.

4. A tampon according to any one of claims 1 to 3, wherein
the first water-soluble carrier has a melting point higher than body temperature, and
the second water-soluble carrier has a melting point lower than or equal to body temperature.

5. A manufacturing method for a tampon having an absorbent body that absorbs liquid, comprising:
obtaining the absorbent body by compressing and shaping an absorbent-body material;
applying onto an outer surface of the absorbent body a first agent that is melted and includes an active pharmaceutical ingredient and a first water-soluble carrier carrying the active pharmaceutical ingredient, the outer surface having a temperature lower than or equal to a freezing point of the first water-soluble carrier; and
applying over the first agent a second agent that is melted and includes an active pharmaceutical ingredient and a second water-soluble carrier that carries the active pharmaceutical ingredient and has a melting point lower than that of the first water-soluble carrier, the first agent having a temperature lower than or equal to a freezing point of the second water-soluble carrier.

6. A manufacturing apparatus for a tampon having an absorbent body that absorbs liquid, comprising:
a compressing-shaping unit with which the absorbent body is obtain by compressing and shaping an absorbent-body material; and
an applying unit
that applies onto an outer surface of the absorbent body a first agent that is melted and includes an active pharmaceutical ingredient and a first water-soluble carrier carrying the active pharmaceutical ingredient, the outer surface having a temperature lower than or equal to a freezing point of the first water-soluble carrier, and
that applies over the first agent the second agent that is melted and includes a active pharmaceutical ingredient and a second water-soluble carrier that carries the active pharmaceutical ingredient and has a melting point lower than that of the first water-soluble carrier, the first agent having a temperature lower than or equal to a freezing point of the second water-soluble carrier.
